# EUROPEAN PATENT APPLICATION

(11) **EP 4 817 558 A1**
(43) Date of publication of application: **07.10.2026**
(21) Application number: 24897626.8
(22) Date of filing: 28.11.2024
(51) Int. Cl.: A61C 13/00, A61C 13/083

(54) **DENTAL BLANK AND METHOD FOR RECOVERING SCRAPS FROM DENTAL BLANKS**

(30) Priority: 29.11.2023 JP 2023201431; 29.11.2023 JP 2023201448
(71) Applicant: Tosoh Corporation, Shunan-shi, Yamaguchi 746-8501 (JP)
(72) Inventor: TODOKO Shigehisa, Tokyo 104-8467 (JP); MACHIDA Kairi, Tokyo 104-8467 (JP)
(74) Representative: Müller-Boré & Partner Patentanwälte PartG mbB
(86) International application number: PCT/JP2024/042085
(87) International publication number: WO 2025/115942

(57) **Abstract**

Provided are a dental blank and a method for collecting scrap materials of a dental blank, described as follows, which allow scrap materials of the dental blank resulting from a milling process to be efficiently and smoothly collected and processed by a recycling process that uses horizontal recycling. The dental blank is provided with an information tag containing at least identification information associated with material information, wherein the identification information is information agreed upon between a manufacturer of a raw material for the dental blank and a manufacturer of the dental blank, and the information tag is an information medium from which the identification information is read in a case where scrap materials of the dental blank are collected. The method for collecting scrap materials of a dental blank comprises a collection step of collecting, from a process operator, scrap materials resulting from a milling process performed on the dental blank; and a selection step of selecting a specific scrap material, which is at least one of the scrap materials and includes an information tag from which the identification information associated with the material information of the dental blank agreed upon between the manufacturer of the raw material for the dental blank and the manufacturer of the dental blank is readable.

## Description

### Technical Field

The present disclosure relates to a dental blank, a dental blank made of a ceramic calcined body and a dental blank that is made of a ceramic calcined body and allows scrap materials resulting from a milling process to be smoothly collected and processed by a recycling process.

### Background Art

Sintered bodies (ceramic materials) made of alumina, zirconia or the like have excellent biocompatibility and are widely used as dental materials. Among ceramics, zirconia sintered bodies have aesthetic properties and high mechanical strength similar to those of natural teeth and are, therefore, frequently used in dental prostheses, such as crowns, bridges, inlays, onlays and abutments.

A method for preparing a dental prosthesis made of a sintered body is as follows. First, a raw material powder is molded to form a green body (green compact), and then the green body is calcined to prepare a calcined body (dental blank). Next, the calcined body is subjected to a milling process in a dental laboratory or the like to form the calcined body into a shape of a dental prosthesis, with the shape being configured taking into account thermal shrinkage caused by sintering. Subsequently, the calcined body is sintered to form a sintered body (dental prosthesis), which is then fine-tuned and eventually attached to a patient.

Incidentally, there is a growing demand to recycle ceramic materials in order to achieve a reduction in industrial waste, a reduction in environmental impact, and the like. There are known methods for recycling a ceramic material, and examples of the methods include a technique for recycling an electrolyte sheet for fuel cells made of a zirconia sintered body (Patent Literature 1) and a technique for recycling grinding balls made of a zirconia sintered body (Patent Literature 2).

### Citation List

### Patent Literature

PTL 1: Japanese Unexamined Patent Application Publication No. 2010-027358
PTL 2: Japanese Unexamined Patent Application Publication No. 10-218662

### Summary of Invention

### Technical Problem

It is believed that calcined bodies, including dental blanks, are suitable for recycling (in particular, horizontal recycling or closed-loop recycling), because they have not yet undergone a sintering process. Unfortunately, Patent Literature 1 and 2 both relate to a technique for recycling a sintered body, that is, they do not relate to the technique for recycling a dental blank, in particular, recycling scrap materials of dental blanks resulting from the milling process.

Furthermore, regarding the dental blank, conforming to the properties of a desired dental prosthesis requires using a plurality of types of dental blanks. Scrap materials of dental blanks resulting from the milling process are a mixture of materials of different compositions and types, and, therefore, collecting such scrap materials for the purpose of recycling is difficult in itself. For this reason, currently, such scrap materials are disposed of as waste by the process operator, such as a dental laboratory, or at best, such scrap materials may be used in cascade recycling, for example, by repurposing them as raw materials for cement. Even if the scrap materials of dental blanks are successfully collected, it is essential, in the case of horizontal recycling, that composition analysis be performed on each of the collected scrap materials before the scrap materials are separated. Thus, horizontal recycling of scrap materials of dental blanks has been practically impossible.

An object of the present disclosure is to provide a dental blank that allows scrap materials of the dental blank resulting from a milling process to be efficiently and smoothly collected and processed by a recycling process that uses horizontal recycling.

### Solution to Problem

Specifically, the present invention is as described in the claims, and a summary of the present disclosure is as follows.
[1] A dental blank provided with an information tag containing at least identification information associated with material information, wherein the identification information is information agreed upon between a manufacturer of a raw material for the dental blank and a manufacturer of the dental blank, and the information tag is an information medium from which the identification information is read in a case where scrap materials of the dental blank are collected.
[2] The dental blank according to [1], wherein the identification information contains one or more selected from the group of raw material ratios, a composition, an average composition, constituent elements, a shade, a shade code, an average shade and an average shade code regarding the dental blank.
[3] The dental blank according to [1] or [2], wherein the identification information contains one or more selected from the group of a production lot, a place of production, a year of production and a collection deadline regarding the dental blank.
[4] The dental blank according to any one of [1] to [3], wherein the identification information contains at least one of a model number and a name of the manufacturer of the dental blank.
[5] The dental blank according to any one of [1] to [4], wherein the dental blank is provided with two or more information tags.
[6] The dental blank according to any one of [1] to [5], wherein the information tag is provided on a non-milled portion of the dental blank.
[7] The dental blank according to any one of [1] to [6], wherein the dental blank has a disc shape, and the information tag is provided on a peripheral side surface of the disc body.
[8] The dental blank according to any one of [1] to [6], wherein the dental blank has a columnar shape, and the information tag is provided on a bottom side of the block body.
[9] The dental blank according to any one of [1] to [8], wherein the dental blank is a ceramic calcined body.
[10] The dental blank according to any one of [1] to [9], wherein the dental blank is a zirconia calcined body.
[11] A method for collecting scrap materials of a dental blank, the method comprising a collection step of collecting, from a process operator, scrap materials resulting from a milling process performed on the dental blank according to any one of [1] to[10]; and a selection step of selecting a specific scrap material, which is at least one of the scrap materials and includes an information tag from which the identification information associated with the material information of the dental blank agreed upon between the manufacturer of the raw material for the dental blank and the manufacturer of the dental blank is readable.
[12] The method for collecting scrap materials of a dental blank according to [11], further comprising a supplying step of supplying a raw material for the dental blank to the blank manufacturer prior to the collection step.
[13] The method for collecting scrap materials of a dental blank according to [12], further comprising an identification information agreement step of making an agreement regarding the identification information prior to the supplying step.
[14] The method for collecting scrap materials of a dental blank according to any one of [11] to [13], further comprising a recycling step of subjecting the specific scrap material to a recycling process based on the identification information after the selection step.

### Advantageous Effects of Invention

The present disclosure can provide a dental blank that allows scrap materials of the dental blank resulting from a milling process to be efficiently and smoothly collected and processed by a recycling process that uses horizontal recycling.

### Brief Description of Drawings

[Fig. 1] Fig. 1 is a diagram illustrating a dental blank 10, according to an embodiment of the present disclosure.
[Fig. 2] Fig. 2 is a cross-sectional view illustrating layers (shades) of the dental blank 10.
[Fig. 3] Fig. 3 is a diagram illustrating a dental block 20.
[Fig. 4] Fig. 4 is a diagram illustrating an information tag 12.
[Fig. 5] Fig. 5 is a diagram illustrating a scrap material 15 of the dental blank 10.
[Fig. 6] Fig. 6 is a flowchart illustrating a method for collecting scrap materials according to an embodiment of the present disclosure.

### Description of Embodiments

A dental blank of the present disclosure will be described below with reference to an example of an embodiment. Any of the various features disclosed in the following description may be combined with another feature, and any combination of the upper limits, lower limits and ranges of numerical values disclosed in this specification is possible.

### [Dental Blank]

The dental blank of the present embodiment will be described below with reference to an example in which the dental blank is a dental blank made of a ceramic calcined body (ceramic blank) or a dental blank made of a zirconia calcined body (zirconia blank).

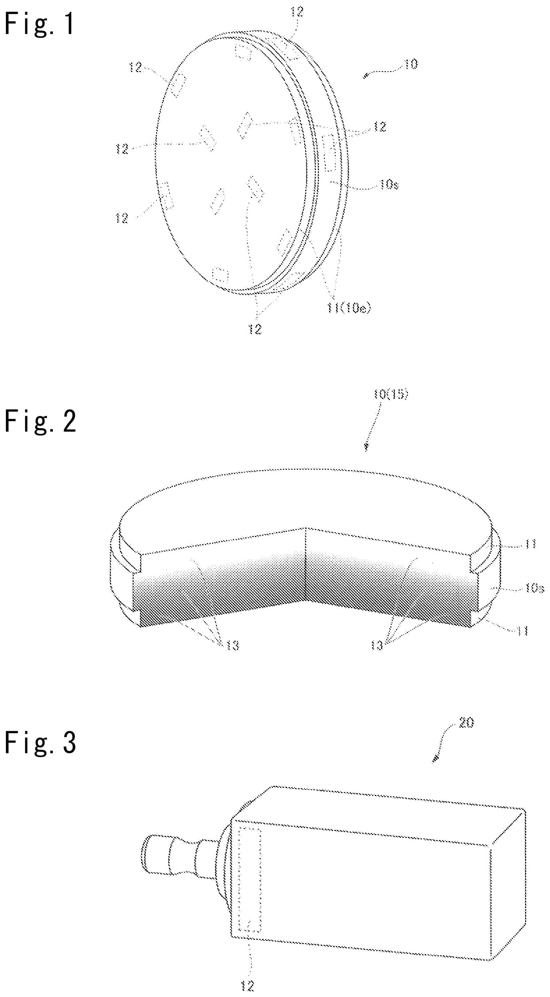

Fig. 1 is a diagram illustrating a dental blank of the present embodiment, which is a dental blank having a disc shape (hereinafter also referred to as a "dental disc").
Fig. 2 is a cross-sectional view of a dental blank 10 formed of a plurality of layers. In Fig. 2, the dental blank is illustrated as a dental disc having a structure in which two or more layers (e.g., 2 or more and 15 or fewer layers, 2 or more and 10 or fewer layers or 3 or more and 8 or fewer layers) with different compositions are disposed; the dental blank 10 is a dental disc having a layered structure in which at least one of a shade and a translucency has a gradient in a thickness direction.
Fig. 3 is a diagram illustrating a columnar dental blank (dental block) 20. Thus, the dental blank 10 may have any shape that allows the dental blank 10 to undergo a CAD/CAM milling process, and examples of the shape include disc shapes, cylindrical shapes, sheet shapes and columnar shapes.

Without limitation, the dental blank 10 may include a pair of peripheral grooves 11. Without limitation, an information tag 12 may be provided on a peripheral side surface 10s. The information tag 12 may be provided at any location that is a non-milled portion (a portion that remains and forms a scrap material 15).

In the case where the dental blank 10 is a dental disc, the dental blank 10 may have any size and, for example, may have a diameter of 10 mm or greater and 100 mm or less and a thickness of 3 mm or greater and 30 mm or less. Preferably, the diameter is 80 mm or greater and 110 mm or less, and the thickness is 12 mm or greater and 20 mm or less. More preferably, the diameter is 98 mm, and the thickness is 16 mm.

Preferably, the dental blank 10 includes a gripping portion (clamping portion) on the peripheral side surface 10s so that securement to a CAD/CAM device can be facilitated. The gripping portion is configured to be gripped by a CAD/CAM device during the milling process. In this case, it is preferable that the dental blank 10 includes the pair of peripheral grooves 11 on peripheries 10e. Utilizing the pair of peripheral grooves 11 enables the dental blank 10 to grip onto a CAD/CAM device. The peripheral side surface 10s is located between the pair of peripheral grooves 11 and is, therefore, a non-milled portion. Note that the dental blank need not include the peripheral grooves 11 provided that the dental blank has a structure that enables securement to a CAD/CAM device for the milling process.

The dental blank 10 is a zirconia blank. This dental blank contains zirconia as a major component and contains, as minor components, a stabilizing element and a coloring element.

The dental blank 10 may have a structure formed of one layer, that is, the dental blank as a whole may have a uniform composition. Meanwhile, dental prostheses need to be in aesthetic harmony with the surrounding tooth structure (natural teeth of the patient). Accordingly, the dental blank 10 may include two or more layers 13 in a thickness direction. In this case, the layers 13 may or may not be clearly distinguished from one another and may include two or more regions (e.g., 2 or more and 15 or fewer regions, 2 or more and 10 or fewer regions or 3 or more and 8 or fewer regions), and the structure may be one in which the two or more layers 13 are disposed such that a gradation is formed. The gradation may be any gradation that is formed by at least one of shades and translucencies. A distinct interface may be present between the regions, or such an interface need not be present. In the case where the dental blank has a structure in which two or more layers are disposed, it is sufficient that the coloring element be included in at least one of the layers.

The dental blank 10 is a calcined body that can be prepared by calcining a green body, which can be prepared by molding a raw material for the dental blank. The dental blank 10 is produced by a manufacturer of the dental blank (disc manufacturer, hereinafter also referred to as a "blank manufacturer"). The raw material for the dental blank is a powder of zirconia (ZrO₂, zirconium dioxide) that contains metal elements, such as a stabilizing element and a coloring element. The stabilizing element may be one or more selected from the group of yttrium (Y), calcium (Ca) and magnesium (Mg) or may be yttrium. The coloring element may be one or more selected from the group of titanium (Ti), iron (Fe), cobalt (Co), nickel (Ni), manganese (Mn), praseodymium (Pr), neodymium (Nd), europium (Eu), gadolinium (Gd), terbium (Tb), erbium (Er) and ytterbium (Yb). Another metal element may be one or more selected from the group of aluminum (Al), silicon (Si) and germanium (Ge). As used herein, the term "zirconia powder" refers to a powder containing zirconia as a major component (typically referred to as a zirconia powder) and is not limited to a powder consisting solely of zirconia. The raw material for the dental blank is produced by a manufacturer of the raw material for the dental blank (hereinafter also simply referred to as a "raw material manufacturer") and supplied to a blank manufacturer. The blank manufacturer molds and calcines the raw material (powder) supplied from the raw material manufacturer, in accordance with the aesthetic properties of a desired dental prosthesis, to produce the dental blank 10 having a predetermined composition (raw material ratios). If necessary, a mixed powder may be formed by mixing two or more raw materials, and the mixed powder may be molded and calcined. Prior to calcination, a solution containing a coloring element may be mixed with the green body.

The dental blank 10 is a precursor of a dental prosthesis and is subjected to a milling process that uses a CAD/CAM device (not illustrated) by a process operator, such as a dental laboratory (dental technician). The milling process mills the dental blank 10 into a shape of a dental prosthesis, such as a crown. The shape resulting from the milling is a shape configured with thermal shrinkage during sintering taken into account, and, therefore, the shape is larger than the shape of the dental prosthesis that is attached to a patient.

The dental blank 10 is provided with an information tag (described below) containing at least identification information associated with material information. The "identification information" is information that can be associated with material information, and the "material information" is information that enables the identification of the dental blank 10. The material information preferably contains at least composition information and more preferably contains one or more pieces of information selected from the group of composition information, product information and production information (each will be described below). Because of the identification information provided on the dental blank 10, efficient selection and collection can be achieved.

Preferably, the identification information contains one or more selected from the group of raw material ratios, the composition (chemical composition), an average composition, constituent elements, the shade, a shade code, an average shade and an average shade code (hereinafter, these pieces of information are also referred to as "composition information"). The composition information enables the properties of the dental blank 10 to be directly ascertained. It is preferable that the identification information contains one or more selected from the group of the raw material ratios, the composition, the constituent elements, the shade and the shade code, or it is preferable that the identification information contains at least one of the composition and the shade code. In the case where the dental blank 10 has a structure formed of two or more layers, it is preferable that the composition information contain one or more selected from the group of the average composition, the average shade and the average shade code, or one or more selected from the group of a representative composition, a representative shade and a representative shade code that can be associated with, for example, a component present in the largest amount; these pieces of information may be contained in addition to the composition, the shade and the shade code, or in place of the composition, the shade and the shade code. The "raw material ratios" are information related to the types (e.g., the product names of the powders) and the amounts of use of the various raw materials used for the production of the dental blank 10. The "shade" is the desired shade of the dental prosthesis that is prepared by sintering the dental blank 10. For example, the shade is indicated by the values of a lightness L*, a chromaticity a* and a chromaticity b* that are represented in the L*a*b* color system. The "shade code" is a code indicating each of the shades in a shade guide, which is, for example, the code represented by any of the letters and numbers, ranging from A1 to D4, of the Vita Classical shade guide.

In cases where the composition information cannot be ascertained, it becomes necessary for the raw material manufacturer to perform component analysis on each of the scrap materials 15. The component analysis that needs to be performed is carried out as follows. A portion of each of the scrap materials 15 is cut, and then, instrumental analysis is performed on a sample solution prepared by dissolving the portion, or instrumental analysis is performed using electron beams or X-rays. In this case, performing component analysis on all the scrap materials requires an enormous amount of effort, and, in addition, in a case where an unexpected element is present, additional time and effort are required for the identification of the component. Accordingly, it is substantially impossible to perform a recycling process using horizontal recycling on these scrap materials even if they are successfully collected.

Preferably, the identification information contains at least one of the model number and the name of the manufacturer of the dental blank (hereinafter, these pieces of information are also referred to as "product information"), or preferably, the identification information contains at least the name of the manufacturer. The "model number" is the product name of a dental blank for the blank manufacturer. In the case where the identification information contains product information, rough separation after collection is made possible. Specifically, the scrap materials collected from dental laboratories and the like may include products of different block manufacturers. In such a case, separation for each manufacturer or separation for each manufacturer and product is made possible, which facilitates efficient separation for horizontal recycling.

Preferably, the identification information contains one or more selected from the group of a production lot, a place of production, a year of production and a collection deadline of the dental blank (hereinafter, these pieces of information are also referred to as "production information"). The identification information more preferably contains at least one of the year of production and the collection deadline and even more preferably contains the collection deadline. In the case where the production information is included, not only recycling is made possible, but also traceability is made possible. As will be described below, the identification information is agreed upon in advance between the raw material manufacturer and the blank manufacturer. Thus, in the case where the collection deadline agreed upon between them is provided, the tracking of not only the dental blank but also the raw materials that were used is made possible. The "year of production" is information from which the time of production can be identified. The year of production may be replaced by a month and year of production or a date of production.

The identification information associated with the material information is information that is used for the collection of the scrap materials 15 of the dental blank 10 and the recycling of the scrap materials 15. That is, the identification information associated with the material information contains information necessary and important for the recycling of the scrap materials 15.

Process operators, such as dental laboratories, mill several different types of dental blanks. Accordingly, the scrap materials 15 that are generated have a wide variety of manufacturers, compositions, constituent elements, shades and the like. If the identification information associated with the material information of the scrap materials 15 (and the dental blanks 10) cannot be ascertained, the recycling process for the scrap materials 15 (dental blanks) performed by an operator belonging to a different entity than the raw material manufacturer or the blank manufacturer becomes very cumbersome, which makes horizontal recycling thereof very difficult.

### [Information Tag]

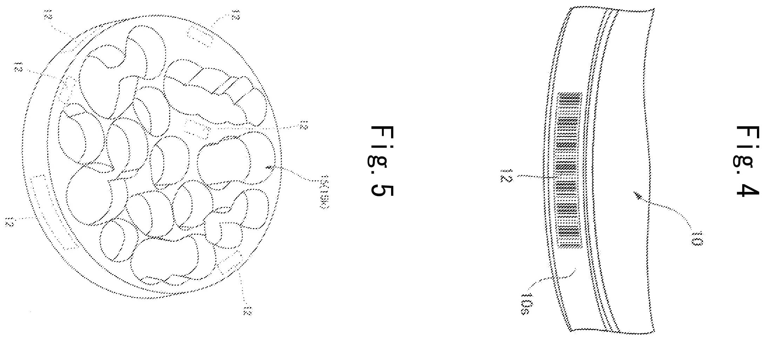

Fig. 4 is a diagram illustrating the information tag 12. The information tag 12 is an information medium from which the identification information is read in a case where the scrap materials 15 of the dental blank 10 are collected. The information tag 12 is provided on the peripheral side surface 10s of the dental blank 10. The information tag 12 may be any information medium from which the identification information associated with the material information can be ascertained. Preferably, the information tag 12 is a machine-readable information medium, and examples thereof include code symbols. The information tag 12 may contain one or more selected from the group of letters, numbers and symbols. The type, size, format and the like of the code symbol may be appropriately selected in accordance with, for example, the amount of information provided to the information tag 12 or the shape of the dental blank 10. For example, the code symbol may be any code symbol that is at least one of a one-dimensional code symbol and a two-dimensional code symbol, specifically, at least one of a bar code and a QR code (registered trademark). The information tag 12 may contain a unique code and the like. Preferably, the code symbol is one from which information is readable even when the code symbol is partially damaged.

Preferably, the information tag 12 is formed of at least one of printed characters and engraved characters. The information tag 12 may be provided by at least one of printing and embossing/debossing. Preferably, the information tag 12 is formed of a material that can be removed by a recycling process, such as printed ink, which may be made from, for example, an organic component, or the information tag 12 is formed by means that can be removed by a recycling process.

It is sufficient that the dental blank 10 be provided with one or more information tags 12. Preferably, the dental blank 10 is provided with two or more information tags 12. Thus, even in a case where some of the tags are damaged or in a case where a scrap material 15 is broken and separated (divided) into several pieces, the remaining information tags 12 facilitate the ascertaining of the identification information of the divided pieces. The number of information tags 12 provided on the dental blank 10 may be 2 or greater and 50 or less or 2 or greater and 10 or less. For the multiple information tags 12, the same code symbol may be used for all of them; furthermore, a bar code and a QR code may be used in combination, or other different types of two or more code symbols may be used in combination.

Preferably, the information tag 12 is provided on a non-milled portion of the dental blank. In this case, damage to the information tag 12 due to the milling process is unlikely to occur. The non-milled portion is a portion or portions of the dental blank 10 that remain after the milling process. For example, in the case of the dental disc, the non-milled portion may be the peripheral side surface 10s, and, in the case of a dental block 20, the non-milled portion may be a bottom side of the block body. Accordingly, in the dental disc, the information tag 12 is preferably disposed at least on the peripheral side surface 10s and more preferably disposed on the peripheral side surface 10s; even more preferably, the information tags 12 are equidistantly disposed on the peripheral side surface 10s. For example, when the number of information tags 12 provided on the peripheral side surface 10s is two, the distance may be 180°; when the number is three, the distance may be 120°; and when the number is four, the distance may be 90°.

It is sufficient that the information tag 12 be provided on at least the non-milled portion. That is, the information tag 12 may be provided on a major surface 10a of the dental blank 10 or may be provided on at least one of the peripheral side surface 10s and the major surface 10a.

In the case where the information tag 12 is provided on the major surface 10a, it is preferable that the information tag 12 be disposed on a portion that remains after the milling process has been performed several times, and the portion may be, for example, at least one of an outer peripheral edge or a middle of the major surface 10a.

### [Scrap Material 15]

Fig. 5 is a diagram illustrating the scrap material 15 of the dental blank 10. A shape (not illustrated) of a dental prosthesis, which is a shape configured with thermal shrinkage taken into account, is milled from the dental blank 10 by using a milling process with a CAD/CAM device. Accordingly, the portion of the dental blank left in the CAD/CAM device as a result of the milling corresponds to the scrap material 15. That is, the scrap material 15 is at least one of a dental blank having one or more holes (milled holes) corresponding to the shape of a dental prosthesis, and fragments of such a dental blank.

### [Method for Collecting Scrap Materials]

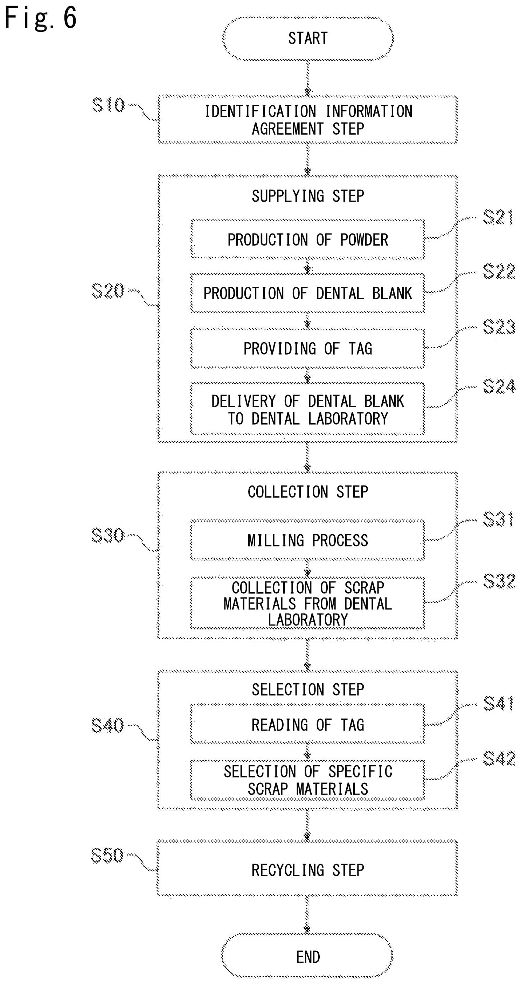

Fig. 6 is a flowchart illustrating a method for collecting scrap materials.

The method for collecting scrap materials of the dental blank of the present embodiment comprises a collection step S30 and a selection step S40. The collection step S30 is a step of collecting, from a process operator, scrap materials 15 resulting from a milling process performed on the dental blank 10, which is provided with the information tag 12 containing at least identification information associated with material information. The selection step S40 is a step of selecting a specific scrap material 15K, which is at least one of the scrap materials 15 and includes an information tag 12 from which the identification information associated with the material information of the dental blank 10 agreed upon between the manufacturer of the raw material for the dental blank and the manufacturer of the dental blank is readable. Additionally, a supplying step S20 may be included, which is a step of supplying a raw material for the dental blank to a blank manufacturer prior to the collection step S30. Furthermore, an identification information agreement step S10 may be included, which is a step of, prior to the supplying step S20, making an agreement between the raw material manufacturer and the blank manufacturer regarding identification information associated with the composition information of the dental blank. Additionally, a recycling step S50 may be included, which is a step of, after the selection step S40, performing a recycling process on the specific scrap material 15K based on the identification information.

The identification information agreement step S10 is a step of making an agreement between the raw material manufacturer and the blank manufacturer regarding the identification information associated with the composition information of the dental blank. In this step, an agreement on the providing of the identification information necessary for the selection and collection of the scrap materials 15 is made. Examples of the identification information include the details of the identification information associated with the material information of the dental blank 10, rules for providing the details and the form of the information tag 12. The identification information may contain a product lineup of the blank manufacturer and other secret information regarding the production by the blank manufacturer. Accordingly, it is preferable that the identification information be managed by the raw material manufacturer and the blank manufacturer as trade secret information for both of them, and it is preferable that the identification information be managed and protected as non-public information.

The supplying step S20 is a step of supplying a raw material for the dental blank to the blank manufacturer and includes a raw material supplying step S21, a dental blank production step S22, a tag providing step S23 and a delivery step S24.

The raw material supplying step S21 is a step in which the raw material manufacturer supplies a raw material for the dental blank to the blank manufacturer. The raw material manufacturer supplies one or more raw materials, preferably a zirconia powder, to the blank manufacturer. Preferably, the powder contains zirconia as a major component and contains, as minor components, a stabilizing element and a coloring element.

In the dental blank production step S22, the blank manufacturer produces a desired dental blank by using the raw material (zirconia powder) supplied from the raw material manufacturer. For the production of the dental blank, the blank manufacturer, if necessary, may mix two or more raw materials together and may use a coloring component, which may be a component other than a powder and may be a coloring solution or the like.

In the tag providing step S23, the blank manufacturer provides the information tag 12 containing identification information to the dental blank 10 or the like. The information tag 12 is provided to the dental blank 10 or the like by means of, for example, at least one of printing and engraving. It is sufficient that the information tag 12 be provided to the dental blank 10 in accordance with the details determined in the above-described identification information agreement step S10, such as the form and the number of information tags 12 and the locations to which the information tags 12 are provided.

The delivery step S24 is a step in which the blank manufacturer delivers the dental blank 10 or the like including the information tags 12 to a dental laboratory or a different process operator that performs the milling process.

The collection step S30 is a step of collecting, from the process operator, scrap materials 15 resulting from a milling process performed on the dental blank 10, which is provided with the information tags 12 containing at least the identification information associated with the material information. The collection step S30 includes a milling process S31 and a scrap material collection step S32.

The milling process S31 is a step of subjecting the dental blank 10 to a milling process. The process operator, such as a dental laboratory, mills the dental blank 10 into a shape of a dental prosthesis. The milling is performed with a processing device, such as a CAD/CAM device, based on information provided by a dentist. Accordingly, a precursor of the dental prosthesis and the scrap materials 15 are generated.

In the scrap material collection step S32, the scrap materials 15 are collected from the process operator, such as a dental laboratory. The collection of the scrap materials 15 may be performed in any manner that enables the scrap materials 15 to be eventually delivered to an operator that performs a recycling process (recycling) on the scrap materials 15. The collection may be performed by at least one of the blank manufacturer and the raw material manufacturer. The scrap materials 15 may be collected by the blank manufacturer and may then be collected by the raw material manufacturer. Alternatively, the scrap materials 15 may be collected by a collection operator that initially collects the scrap materials 15 from the process operator. The collection operator may be, for example, a collection operator designated by at least one of the blank manufacturer and the raw material manufacturer. Thus, the collection in the collection step S30 may be direct collection from the process operator or may be collection via a collection operator instead of or in addition to the process operator; the collection operator is an operator that initially collects the scrap materials 15 from the process operator.

Alternatively, the scrap materials 15 may be sent to at least one of the blank manufacturer and the raw material manufacturer by the process operator and, accordingly, may be collected.

Since the scrap material 15 is a calcined body having a milled hole, the scrap material 15 is very brittle and prone to breakage. Because of this, there is a possibility that, during transportation or the like in the scrap material collection step S32, the scrap materials 15 may be broken (destroyed) and divided into two or more fragments (scrap material pieces, broken fragments).

The selection step S40 is a step of selecting the specific scrap material 15K, which is at least one of the scrap materials 15 and includes an information tag 12 from which the identification information associated with the material information of the dental blank 10 agreed upon between the manufacturer of the raw material for the dental blank and the manufacturer of the dental blank is readable. The selection step S40 includes a tag reading step S41 and a scrap material selection step S42.

In the tag reading step S41, the information tags 12 of all the collected scrap materials 15 are read. In the case of a scrap material 15 that has been divided into fragments, it is sufficient that the reading of the information tag 12 be performed on fragments including the information tag 12.

The reading of the information tag 12 may be performed by any means that enables the identification information to be ascertained. For example, the reading can be performed by using a code symbol reading means, specifically, by using at least one of a bar code reader and image analysis.

In the scrap material selection step S42, the scrap material 15K from which identification information is readable through the information tag 12 is selected and separated (hereinafter, this scrap material is also referred to as a "specific scrap material"). Since the properties of the specific scrap material 15K, such as the material composition, are known, the specific scrap material 15K can be collected and suitably used in horizontal recycling.

It is sufficient that the specific scrap material 15K be selected and separated based on its properties. Preferably, the specific scrap material 15K is selected and separated based on at least one of the constituent elements and the composition. In this case, the specific scrap material 15K can be more easily used in a recycling process (recycling).

In the case of scrap materials 15 or their fragments from which identification information is not readable because of, for example, damage to the information tag 12, loss of the information tag 12, or the like, their material information cannot be ascertained. Such scrap materials can be selected and separated by acquiring their composition information separately by instrumental analysis or the like; however, from the standpoint of process efficiency, they may be handled as materials that are used in cascade recycling or are disposed of as waste.

The recycling step S50 is a step of subjecting the specific scrap material 15K to a recycling process (recycling) that is performed based on the identification information. The recycling process can be performed on the specific scrap material 15K based on the identification information. Since the properties of the specific scrap material 15K, such as the material composition, are known, it is possible to set appropriate recycling conditions for each of the groups of selected specific scrap materials 15K, based on the identification information.

A recycling method for the recycling step S50 may be a recycling method in which the specific scrap material 15K is ground to be powdered to form a powder (recycled powder) (hereinafter, this method is also referred to as a "grinding recycling method"). If necessary, the recycling step S50 may include at least one of the following steps (none are illustrated): a step of cleaning at least one of the specific scrap material 15K and its ground product prior to grinding, to remove impurities: a particle size adjustment step for adjusting a particle size of the ground product of the specific scrap material 15K after grinding; and a step of drying at least one of the specific scrap material 15K and its ground product.

The powder prepared by the grinding recycling method (recycled powder) can directly serve as a raw material for calcined bodies and sintered bodies. The recycled powder has a composition derived from the dental blank 10. Thus, the recycled powder is particularly suitable as a raw material for a dental blank (recycled dental blank).

The recycling method for the recycling step S50 may be a recycling method in which the specific scrap material 15K is dissolved and liquefied to form a solution (recycled solution) (hereinafter, this method is also referred to as a "dissolution recycling method"). The dissolution can be carried out by contacting the specific scrap material 15K with a solution that can dissolve the specific scrap material 15K, such as acid. The specific scrap material 15K may be ground, cleaned and dried, before being dissolved. The solution (recycled solution) prepared by the dissolution recycling method can serve as a raw material solution for the synthesis of a powder, preferably as a raw material solution for the synthesis of a zirconia powder. The solution can serve as a raw material for one or more methods for producing a powder selected from the group of neutralization co-precipitation, hydrolysis and a hydrothermal treatment. The thus-prepared raw material is more suitable as a raw material for dental blanks.

Furthermore, the recycled solution may be subjected to ion exchange or the like to refine an element present therein, to form various metal ion solutions, including a zirconium solution, to be used in a desired application.

As described, the dental blank of the present embodiment enables efficient collection of the scrap materials 15 of the dental blank 10, to achieve horizontal recycling that allows the scrap materials 15 to be used as a material for dental blanks.

The above description describes a zirconia block as an example of a preferred embodiment of the present disclosure. Note that in the present disclosure, the dental blank may be any dental blank that is a ceramic calcined body and may be a dental blank made of a calcined body of a metal oxide, such as alumina.

The entire contents of the specifications, claims, drawings and abstracts of Japanese Patent Application No. 2023-201431, filed on November 29, 2023, and Japanese Patent Application No. 2023-201448, filed on November 29, 2023, are cited and incorporated herein as a disclosure of the specification of the present disclosure.

### Reference Signs List

- 10: dental disc (dental blank)
- 10e: periphery
- 10s: peripheral side surface (non-milled portion)
- 11: peripheral groove
- 12: tag (code symbol)
- 15: scrap material
- 15K: specific scrap material
- 20: dental block (dental blank)

## Claims

1. A dental blank provided with an information tag containing at least identification information associated with material information, wherein
the identification information is information agreed upon between a manufacturer of a raw material for the dental blank and a manufacturer of the dental blank, and
the information tag is an information medium from which the identification information is read in a case where scrap materials of the dental blank are collected.

2. The dental blank according to claim 1, wherein the identification information contains one or more selected from the group of raw material ratios, a composition, an average composition, constituent elements, a shade, a shade code, an average shade and an average shade code regarding the dental blank.

3. The dental blank according to claim 1 or 2, wherein the identification information contains one or more selected from the group of a production lot, a place of production, a year of production and a collection deadline regarding the dental blank.

4. The dental blank according to claim 1 or 2, wherein the identification information contains at least one of a model number and a name of the manufacturer of the dental blank.

5. The dental blank according to claim 1 or 2, wherein the dental blank is provided with two or more information tags.

6. The dental blank according to claim 1 or 2, wherein the information tag is provided on a non-milled portion of the dental blank.

7. The dental blank according to claim 1 or 2, wherein the dental blank has a disc shape, and the information tag is provided on a peripheral side surface of the disc body.

8. The dental blank according to claim 1 or 2, wherein the dental blank has a columnar shape, and the information tag is provided on a bottom side of the block body.

9. The dental blank according to claim 1 or 2, wherein the dental blank is a ceramic calcined body.

10. The dental blank according to claim 1 or 2, wherein the dental blank is a zirconia calcined body.

11. A method for collecting scrap materials of a dental blank, the method comprising:
a collection step of collecting, from a process operator, scrap materials resulting from a milling process performed on the dental blank according to claim 1; and
a selection step of selecting a specific scrap material, which is at least one of the scrap materials and includes an information tag from which the identification information associated with the material information of the dental blank agreed upon between the manufacturer of the raw material for the dental blank and the manufacturer of the dental blank is readable.

12. The method for collecting scrap materials of a dental blank according to claim 11, further comprising a supplying step of supplying a raw material for the dental blank to a blank manufacturer prior to the collection step.

13. The method for collecting scrap materials of a dental blank according to claim 12, further comprising an identification information agreement step of making an agreement regarding the identification information prior to the supplying step.

14. The method for collecting scrap materials of a dental blank according to claim 11 or 12, further comprising a recycling step of subjecting the specific scrap material to a recycling process based on the identification information after the selection step.
